(19) Europäisches Patentamt
European Patent Office
Office européen des brevets

(11) Publication number: **0 173 544 B1**

# (12) EUROPEAN PATENT SPECIFICATION

(45) Date of publication of patent specification: **22.05.91**

(51) Int. Cl.5: **C07C 29/76**, C07C 31/04, C07C 31/08

(21) Application number: **85305942.6**

(22) Date of filing: **21.08.85**

## (54) Process for the recovery of a low molecular weight alcohol from an aqueous fermentation medium.

(30) Priority: **22.08.84 AU 6711/84**

(43) Date of publication of application:
**05.03.86 Bulletin 86/10**

(45) Publication of the grant of the patent:
**22.05.91 Bulletin 91/21**

(84) Designated Contracting States:
**AT BE CH DE FR GB IT LI LU NL SE**

(56) References cited:
**EP-A- 0 097 461**

(73) Proprietor: **APACE RESEARCH LIMITED**
**Hawkesbury Agricultural College**
**Richmond New South Wales(AU)**

(72) Inventor: **Reeves, Russell Robert**
**C/o Hawkesbury Agricultural College**
**Richmond, New South Wales(AU)**

(74) Representative: **Spencer, Graham Easdale et al**
**Northumberland House 303-306 High Holborn**
**London WC1V 7LE(GB)**

EP 0 173 544 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is concerned with a process for the recovery of a low molecular weight alcohol from an aqueous fermentation medium.

The conventional method for concentrating low molecular weight alcohols (for example methanol and ethanol) from dilute aqueous solutions of these alcohols is by distillation. Using conventional distillation techniques, the concentration of ethanol, for example, which can be achieved, is 96.5% by volume, at which stage an azeotropic mixture of water and ethanol is formed. The preparation of anhydrous ethanol requires further azeotropic distillation with cyclohexane or benzene.

For laboratory preparations and other applications where energy balance considerations are not important, distillation has proved satisfactory. However, when ethanol is produced for use as a fuel it is desirable to use as little processing energy as possible so that considerably more energy is obtained in the form of ethanol than is put in to the process as heat or electricity. These energy considerations are usually expressed in the form of an energy ratio, defined as the energy available in the ethanol divided by the process energy used to obtain the ethanol. For current processes of ethanol production, this figure varies from 1.5 for a starch raw material 2.2 for a sugar raw material. These figures decrease to 1.0 and 1.3 respectively when account is taken of the energy required for the evaporative treatment of the effluent waste from both the fermentation and distillation processes.

Two major drawbacks to a satisfactory fuel ethanol industry are therefore the high processing energy requirement of conventional technology (in particular for the distillation step) and the waste disposal problem. Thus two of the key objectives in the development of any process for ethanol production by fermentation are to reduce the energy requirements of the process to attain a satisfactory net energy balance and to ensure that any waste has virtually no environmental impact.

Currently there is a world-wide interest in the development of methods other than distillation for the recovery of ethanol from fermented biomass. Methods under investigation include membrane filtration and gel filtration techniques, as well as solvent extraction. It is also widely acknowledged that satisfactory treatment of the effluent waste from the fermentation (and distillation) process for ethanol production is of considerable importance. Any method which combines the two processes in a single step is therefore of great commercial interest.

In the conventional fermentation-distillation process for producing ethanol, starch or sugar is fermented by yeasts or bacteria to produce a solution of ethanol and other organic materials in water (usually 8-10% v/v ethanol) which is then concentrated by distillation to give 95-100% v/v ethanol. The process of the present invention provides a low energy alternative to distillation for the recovery of ethanol.

According to a preferred embodiment, therefore, the process of the invention is used for the recovery of ethanol from fermented biomass, for example in an integrated ethanol-water separation/fermentation effluent waste treatment process.

The major waste from a distillery is the effluent from the first or stripping column. It is known as dunder, stillage, slops, vinasse or vinhoto. The composition depends to some extent on the nature of the substrate. Some figures are given in the following Table:

DISTILLERY WASTE COMPOSITION

| Substrate: Dunder composition | Molasses | Cane Juice |
|---|---|---|
| pH | 4.8 | 3.7 - 5.9 |
| s.g. | 1.05 | |
| BOD mg/L | 45,000 | 20,000 |
| COD mg/L | 113,000 | |
| Dissolved solids % | 10 | |
| Suspended solids % | 1 | |
| Total solids | 11 | 6 - 11 |
| Ash % | 3 | 2 - 3 |
| Organic matter % | 8 | 4.6 - 8 2.9 |

Data on dunder from molasses are more readily available than data on the dunder of other substrates. Molasses dunder is a rich-brown to black colour with a pleasant smell. There is little precise knowledge of its composition, but it comprises the non-fermentable substances of the substrate, yeast metabolites and yeast cell contents. These include gums and many substances of plant origin.

Disposal of dunder is a serious problem. A 50,000 Kl/annum molasses distillery produces an effluent with a B.O.D. equivalent to the domestic sewage from a city of 1 million people. If ethanol is to become widely used as a fuel, there needs to be a satisfactory solution to waste disposal.

The possible disposal options are:

(1) discharge into streams;
(2) discharge to sewer;
(3) discharge into the sea;
(4) aerobic digestion;
(5) anaerobic digestion;
(6) submerged combustion;
(7) evaporation and incineration;
(8) ultrafiltration;
(9) stock feed;
(10) raw material for single cell protein usage; and
(11) fertiliser.

Of these the most convenient and environmentally sound approach is to concentrate the dunder to 60% v/v solids by evaporation, and then combust it directly in a special incinerator. The heat of combustion can be used for the evaporation and the ash from the incineration process is a convenient fertiliser, typically comprising from 30% to 40% of potash ($K_2O$) and from 2% to 3% of $P_2O_5$.

Evaporation of the dunder, combined with normal distillation, requires about 6 tonnes of steam per kilolitre of ethanol. About 3 tonnes of this steam can be provided by combusting the dunder solids, leaving only 3 tonnes to be raised by alternative means.

It has previously been reported that salts such as potassium carbonate and copper sulphate can be used as a dehydrating agent for removing small quantities of water from concentrated alcohol (see "Inorganic and Theoretical Chemistry", F. Sherwood Taylor, P287 and "Guide to the Analysis of Alcohols", published by Interscience, page A-13). The use of potassium carbonate to assist in the partitioning extraction of alcohol from blood has also been reported; equal amounts of blood and propyl acetate were mixed with potassium carbonate and the alcohol from the blood partitioned with the propyl acetate (see "Guide to the Analysis of Alcohols", published by Interscience, Pages A-14 and A-15).

In EP-A-97461 a process is described for the recovery of a water-soluble alcohol having more than one carbon atom from an aqueous mixture containing the alcohol, the process comprising the addition to the mixture of at least 0.5% of a base selected from ammonium or alkali or alkaline earth metal hydroxide, ammonium or alkali or alkaline earth metal carbonate or a mixture thereof, and at least 0.2% of an electrolyte, the addition causing the mixture to separate into two immiscible layers which are then separated. Preferred electrolytes for use in this process are stated to be sodium chloride, potassium chloride, sodium sulphate, sodium nitrate, sodium glycolate, glycolic acid, and sodium monochloroacetate.

We have now found that the addition of a base or basic salt to an aqueous solution of a low molecular weight alcohol alters the nature of the water component so that the aqueous component and the alcohol component are no longer miscible and phase separation occurs. This enables the upper alcohol phase to be decanted off and the alcohol recovered therefrom.

According to the present invention, therefore, there is provided a process for the recovery of a low molecular weight alcohol from an aqueous fermentation medium containing not more than 40% by volume of the alcohol, which comprises

dissolving in the fermentation medium at least 26g/100 ml of fermentation medium of a base or basic salt of an acid having a $pK_a$ of more than 6, the base or salt having a solubility of at least 26g/100 ml of fermentation medium and being substantially more soluble in water than in the alcohol, to form (a) an alcohol-rich phase, (b) an alcohol-poor, base or salt-rich phase, and (c) a solid phase of flocculated material that was originally dissolved in the fermentation medium,

recovering the alcohol-rich phase and the solid phase separately, and

recovering the base or salt from the base or salt-rich phase in a form suitable for re-use in this process.

(The symbol $pK_a$ indicates the logarithm of the reciprocal of the dissociation constant of an acid.) The process of the invention can be operated in a batchwise or continuous manner.

The alcohols which may be separated from aqueous fermentation media by the process according to the invention are those having a $pK_a$ of greater than 14, more particularly, methanol and ethanol.

Suitable bases or basic salts for use in the process of the invention are those which are derived from an acid having a $pK_a$ of greater than 6; preferred bases or basic salts include, for example, sodium carbonate, potassium carbonate, sodium citrate, sodium hydroxide, potassium hdyroxide, rubidium carbonate, and cesium carbonate. Anhydrous potassium carbonate, which has a high solubility in water but is substantially insoluble in lower alcohols and other weak organic acids, is particularly preferred - the acid from which potassium carbonate is derived has a $pK_a$ of 10.25.

The phase separation is induced by dissolving the base or basic salt in the water/alcohol solution in an amount of at least 26g/100ml of solution, preferably until the solution is substantially saturated with the base or basic salt. The optimum concentration of the base or basic salt may be determined by simple experimentation; in practice it has been found that a concentration of at least 26g/100ml of solution is required for satisfactory results, preferably at least 100g/100ml. The higher the concentration of base or basic salt in the solution, the less water will the separated organic phase contain. Thus the addition of 26 grams of sodium carbonate per 100ml of an alcohol/water solution induces the separation of an alcohol phase containing from 30% to 40% v/v water, whereas the addition of 100 grams of sodium carbonate per 100ml of solution induces the separation of an alcohol phase containing only from 10% to 20% v/v water.

The base or basic salt for use in the process of the invention should be substantially insoluble in the alcohol so that it essentially dissolves in only the water component of the mixture. In the case of carbonate salts, since both water and bicarbonate ion are considerably stronger acids than aliphatic alcohols, the relevant equilibria are:

$$CO_3^{2-} + H_2O \rightleftharpoons HCO_3^- + OH^- \quad \ldots \quad (1)$$

$$ROH + OH^- \not\rightleftharpoons RO^- + H_2O \quad \ldots \quad (2) \text{ no}$$

reaction where ROH is an aliphatic alcohol.

Separation of the alcohol and aqueous phases occurs because the hydrogen-bonding/protonation mechanism of alcohol/water miscibility, viz:

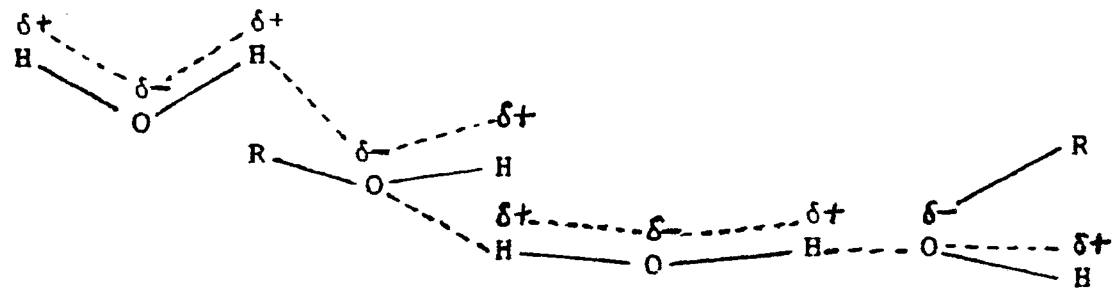

is destroyed by the high concentration of hydroxyl ions. The bicarbonate ion is a stronger acid than ROH so that the reverse of reaction (1) applies and not reaction (2).

The base used to induce the phase separation can be an hydroxide, but hydroxyl salts are less satisfactory because of their relatively high solubility in low molecular weight alcohols. The solubility of the basic compound used to induce the phase separation is an important consideration as sufficient must dissolve in the solution to considerably reduce the effective concentration of water by the interaction of the water with the base. For basic salts other than hydroxide, the higher the $pK_a$ value of the parent acid, the lower the effective concentration of water upon addition of the salt.

When the process of the invention is used in accordance with the preferred embodiment, i.e. for the recovery of ethanol from fermented biomass, flocculation of most of the soluble dunder components occurs; according to one aspect of the embodiment, the flocculated material is removed from the solution and dried to give a solid "dunder cake" which is used as a fuel source.

In order that the invention may be more fully understood, a preferred process for the recovery of ethanol using the process of the invention will now be described with reference to the accompanying drawing, which is a block diagram of a pilot plant for the continuous recovery of ethanol from an aqueous solution by the process of the invention.

To determine the commercial viability of the process of the invention, a pilot plant was constructed in accordance with the block diagram shown in the drawing. As a simulated feedstock, a synthetic ethanol-water mixture was prepared from 95% v/v ethanol (industrial methylated spirits or IMS) and water. This mixture did not, of course, contain any yeasts, other organic materials, or impurities such as would normally be present in a fermented biomass.

With reference to the drawing, the prepared feed solution was fed through inlet line 10 and mixed with potassium carbonate ($K_2CO_3$) in mixing tank 11 until an approximately 50% w/v carbonate solution was obtained. The solution was then pumped from tank 11 through filter 12 to a separation tank 13 at a rate of about 6 litres/min. The ethanolic phase, having a lower specific gravity than the potassium carbonate solution, was decanted into a holding tank 14. The underflow from separation tank 13 was passed through a heat exchanger 15 at a pressure of 25 psi (172 kPa) and heated to a temperature of 93°C. Any remaining alcohol was vaporised in flashing tank 16. The alcohol vapour from the flashing tank 16 was condensed in condenser 17 and was then cooled by cooling water flowing through line 18 and returned to the separation tank 13.

The potassium carbonate solution which remained in flashing tank 16 was pumped through heat exchanger 19, heated by steam admitted through line 21, to a low-pressure, i.e. sub-atmospheric, single stage evaporator 22 operating at a pressure of 2-4 psi(14-28 kPa). Recirculation through heat exchanger 19 allowed the solution to achieve 67% concentration, which was still a pumpable slurry. This concentrated slurry was then pumped back to mixing tank 11 for dilution with fresh feed solution before being recirculated.

In accordance with the preferred embodiment of the invention, the process was then carried out in the pilot plant using feedstocks consisting of fermented biomass solutions of the type obtained from industrial fermentation processes.

When using the process according to the preferred embodiment, the fermented biomass solution is first filtered to remove any solid particulate matter. The base or basic salt is then added. This causes the soluble dunder components to flocculate as a result of the high ionic strength of the resulting solution. The flocculated material is then removed from the solution by filtration or centrifugation and dried to give a solid dunder cake. Losses of the base or basic salt during this step are minimal and directly proportional to the moisture content of the separated dunder cake; there appears to be no complexing of the bases or salts

with the dunder material. After separation of the dunder material, the two liquid phases are obtained as before. The dry dunder cake is used as a fuel source to provide heat for the evaporation of water from the basic phase in order to recover the base or basic salt.

In order that the invention may be more fully understood, the following examples are given by way of illustration only.

Example 1

440g of anhydrous potassium carbonate was added to a solution containing 360ml of water and 40ml of ethanol and the mixture stirred until all of the potassium carbonate had dissolved. The mixture separated into two phases having the following compositions:

| | Ethanol | Water | Potassium Carbonate |
|---|---|---|---|
| Upper layer | ~ 40 mls | ~ 3% v/v | - |
| Lower layer | ~ 0.5 v/v | ~ 360 mls | 440 grams |

The amount of alcohol remaining in the lower aqueous potassium carbonate phase represents the solubility of alcohol in the aqueous phase. This figure decreases with higher concentrations of potassium carbonate. Because the vapour pressure of the alcohol in the potassium carbonate solution is higher than that in a solution of alcohol and water alone, the amount of energy required to recover this remaining alcohol is small.

Example 2

Tables 1A and 1B list further examples which show that the ability of the base or basic salt to induce phase separation is dependent upon the $pK_a$ value of the parent acid and is independent of the ionic strength of the basic compound/water/alcohol solution. Potassium iodide, potassium thiocyanate, and potassium acetate are included in the Tables for comparitive purposes only. It will be seen that only those bases or basic salts derived from acids having a $pK_a$ of greater than 6 are capable of inducing phase separation.

TABLE 1A

PHYSICAL CONSTANTS OF BASE OR BASIC SALTS

| Compound | Solubility grams/100ml Water Cold | Hot | Ethanol | Molecular Weight | $pK_a$ of parent acid |
|---|---|---|---|---|---|
| | (at temperature indicated in °C) | | | | |
| Potassium Iodide (KI) | 127.5(0°) | 208(100°) | 1.88(25°) | 166.01 | 0.77 |
| Potassium Thiocyanate (KNCS) | 177.2(0°) | 217(20°) | soluble | 97.18 | 0.85 |
| Potassium Acetate ($CH_3CO_2K$) | 253(20°) | 492(62°) | 33 | 98.15 | 4.75 |
| Potassium Citrate ($K_3C_6H_5O_7 \cdot H_2O$) | 167(15°) | 199.7(31°) | slightly soluble | 324.42 | 6.39 |
| Potassium Carbonate ($K_2CO_3$) | 112(20°) | 156(100°) | insoluble | 138.21 | 10.25 |
| Potassium Hydroxide (KOH) | 107(15°) | 178(100°) | very soluble | 56.11 | $>14$ |

TABLE 1B

| PHYSICAL CONSTANTS OF SOLUTIONS OF BASE OR BASIC SALTS/WATER/ALCOHOL | | | |
|---|---|---|---|
| Compound | 10% v/v aqueous solution of alcohol to which compound is added | | Volume of separated alcohol phase as (approx) percentage of volume of alcohol in original aqueous solution |
| | Molar concentration of compound | Ionic strength of resultant solution | |
| Potassium Iodide (KI) | 10.00 | 10.00 | 0 |
| Potassium Thiocyanate (KNCS) | 22.64 | 22.64 | 0 |
| Potassium Acetate ($KC_2H_3O_2$) | 22.42 | 22.42 | 0 |
| Potassium Citrate ($K_3C_6H_5O_7 \cdot H_2O$) | 5.24 | 31.44 | <10 |
| Potassium Carbonate ($K_2CO_3$) | 7.96 | 23.88 | 100 |
| Potassium Hydroxide (KOH) | 21.39 | 21.39 | 100 |

Examples 3, 4, and 5 relate to the recovery of ethanol using the pilot plant process described above.

Example 3

An 880 litre batch of the synthetic aqueous ethanol and potassium carbonate was prepared in mixing tank 11. The composition of the solution was as follows:

| | |
|---|---|
| Potassium carbonate | 48.91% w/v |
| Ethanol | 9.975% v/v |

Five hundred litres of the solution was run through the recovery plant at a rate of 6.25 litres/min and the following quantities of ethanol were recovered. (The flash tank 16 was operated cold and used as a separation tank).

| | | |
|---|---|---|
| Quantity of "alcohol" in 14 | = | 49.4 litres |
| Quantity of "alcohol" in 16 | = | 1.437 litres |
| Total quantity of "alcohol" collected | = | 50.837 litres |

The concentration of alcohol recovered was 93.6% v/v corresponding to an equivalent "100% alcohol" content of 50.837 x 0.936 = 47.58 litres.

The initial alcohol content in 500 litres was 500 x 9.975 = 49.875 litres.

Therefore the alcohol recovery was 47.58/49.875 x 100 = 95.4%.

Example 4

73.4 litres of 93.6% (68.7 litres of 100%) ethanol was added to the contents of mixing tank 11, which increased the volume to 807 litres (equivalent to 8.5% v/v ethanol).

The total amount of alcohol recovered from a throughput of 513.8 litres at an average flow rate of 5.7 litres per minute was:

| | |
|---|---|
| Separation tank 14, yield of 96.2% alcohol: | 42.1 litres |
| Flashing tank 16, yield of 96.2% alcohol: | 3.17 litres |

Total yield of 96.2% alcohol: 45.27 litres which is equivalent to 43.45 litres of 100% alcohol.

Percentage of 100% alcohol in 513.8 litres = 8.457%.

Therefore total alcohol recovery was $\frac{8.457}{8.5} \times 100$ = 99.5%.

Example 5

"Dead wash" (the product formed by the complete fermentation of molasses) is a rich dark brown to black coloured liquid obtained from an alcohol distillery and containing approximately 10% of dissolved solids, 1% of suspended solids and 8% v/v ethanol. To recover the ethanol from the dead wash, the suspended solids were first removed by filtration. The filtered dead wash was then mixed in a mixing tank with anhydrous potassium carbonate in a ratio of 115 grams of potassium carbonate to 100ml of dead wash. Upon mixing, the dissolved solids in the dead wash flocculated. In excess of 99% of the flocculated solids were then removed by filtration and dried to give a solid dunder cake.

The separated ethanol phase was light brown in colour as it contained colourants and fusel oils representing weak organic acids with a $PK_a > 14$ which were separated along with the ethanol. The concentration of the separated ethanol phase was approximately 97% v/v ethanol.

## Claims

1. A process for the recovery of a low molecular weight alcohol from an aqucous fermentation medium containing no more than 40% by volume of the alcohol, which comprises
dissolving in the fermentation medium at least 26g/100 ml of fermentation medium of a base or basic salt of an acid having a pKa of more than 6, the base or salt having a solubility of at least 26g/100 ml of fermentation medium and being substantially more soluble in water than in the alcohol, to form (a) an alcohol-rich phase, (b) an alcohol-poor, base or salt-rich phase, and (c) a solid phase of flocculated material that was originally dissolved in the fermentation medium,
recovering the alcohol-rich phase and the solid phase separately, and
recovering the base or salt from the base or salt-rich phase in a form suitable for re-use in this process.

2. A process according to claim 1, in which the low molecular weight alcohol is ethanol.

3. A process according to claim 1 or 2, in which the base or basic salt is potassium carbonate, sodium carbonate, sodium citrate, sodium hydroxide, or potassium hydroxide.

4. A process according to any of claims 1 to 3, in which the fermentation medium is substantially saturated with the base or basic salt.

5. A process according to any of claims 1 to 4, in which solid particulate matter present in the fermentation medium prior to the addition of the base or basic salt thereto, is removed therefrom prior to such addition of base or basic salt, and the solid phase of flocculated material formed after addition of the base or basic salt is removed before separation of the liquid phases.

6. A process according to claim 5, in which the separated flocculated material is used as a fuel source.

## Revendications

1. Procédé de récupération d'un alcool à faible poids moléculaire à partir d'un milieu de fermentation aqueux contenant une quantité inférieure ou égale à 40 % en volume de l'alcool, comprenant
la dissolution dans le milieu de fermentation d'au moins 26 g/100 ml de milieu de fermentation d'une base ou d'un sel basique d'un acide ayant une $pk_a$ supérieure à 6, la base ou le sel ayant une solubilité minimum de 26 g/100 ml de milieu de fermentation et étant sensiblement plus soluble dans l'eau que dans l'alcool, pour former (a) une phase riche en alcool, (b) une phase pauvre en alcool et riche en base ou en sel et (c) une phase solide de matériau floculé originellement dissous dans le milieu de fermentation,
la récupération distincte de la phase riche en alcool et la phase solide et
la récupération de la base ou du sel à partir de la phase riche en base ou en sel sous une forme convenant à la réutilisation dans le présent procédé.

2. Procédé selon la revendication 1, dans lequel l'alcool à faible poids moléculaire est de l'éthanol.

3. Procédé selon la revendication 1 ou 2, dans lequel la base ou le sel basique est du carbonate de

potassium, du carbonate de sodium, du citrate de sodium, de l'hydroxyde de sodium ou de l'hydroxyde de potassium.

4. Procédé selon l'une quelconque des revendications 1 à 3, dans lequel le milieu de fermentation est pratiquement saturé par la base ou le sel basique.

5. Procédé selon l'une quelconque des revendications 1 à 4, dans lequel la substance particulaire solide présente dans le milieu de fermentation avant l'addition à celui-ci de la base ou du sel basique en est retirée avant cette addition de la base ou du sel basique, et la phase solide de matériau floculé formée après l'addition de la base ou du sel basique est retirée avant la séparation des phases liquides.

6. Procédé selon la revendication 5, dans lequel le matériau floculé séparé est utilisé comme source de combustible.

**Ansprüche**

1. Verfahren zur Gewinnung eines Alkohols mit niedrigem Molekulargewicht aus einem wässrigen Fermentationsmedium, enthaltend nicht mehr als 40 Vol.% des Alkohols, das

   das losen von wenigstens 26g/100 ml des Fermentationsmediums einer Base oder eines basischen Salzes einer Säure mit einem $pk_a$ von mehr als 6 in dem Fermentationsmedium wobei die Base oder das Salz eine Löslichkeit von wenigstens 26g/100 ml des Fermentationsmediums besitzt und im wesentlichen in Wasser löslicher als in dem Alkohol ist, zur Bildung (a) einer alkoholreichen Phase, (b) einer alkoholarmen, basen- oder salzreichen Phase und (c) einer festen Phase aus ausgeflocktem Material, das ursprünglich in dem Fermentationsmedium gelöst war,

   das getrennte Gewinnen der alkoholreichen Phase und der festen Phase und

   das Gewinnen der Base oder des Salzes aus der basen- oder salzreichen Phase in einer Form, die zur Wiederverwendung in diesem Verfahren geeignet ist,

   umfaßt.

2. Verfahren nach Anspruch 1, worin der Alkohol mit niedrigem Molekulargewicht Ethanol ist.

3. Verfahren nach Anspruch 1 oder 2, worin die Base oder das basische Salz Kaliumcarbonat, Natriumcarbonat, Natriumcitrat, Natriumhydroxid oder Kaliumhydroxid ist.

4. Verfahren nach einem der Ansprüche 1 bis 3, worin das Fermentationsmedium im wesentlichen mit der Base oder dem basischem Salz gesättigt ist.

5. Verfahren nach einem der Ansprüche 1 bis 4, worin das feste teilchenförmige Material, das in dem Fermentationsmedium vor der Zugabe der Base oder des basischen Salzes vorliegt, daraus vor der Zugabe der Base oder des basischen Salzes entfernt wird und die feste Phase aus ausgeflocktem Material, die sich nach der Zugabe der Base oder des basischen Salzes bildet, vor der Trennung der flüssigen Phasen entfernt wird.

6. Verfahren nach Anspruch 5, worin das getrennte, ausgeflockte Material als Brennstoffquelle verwendet wird.

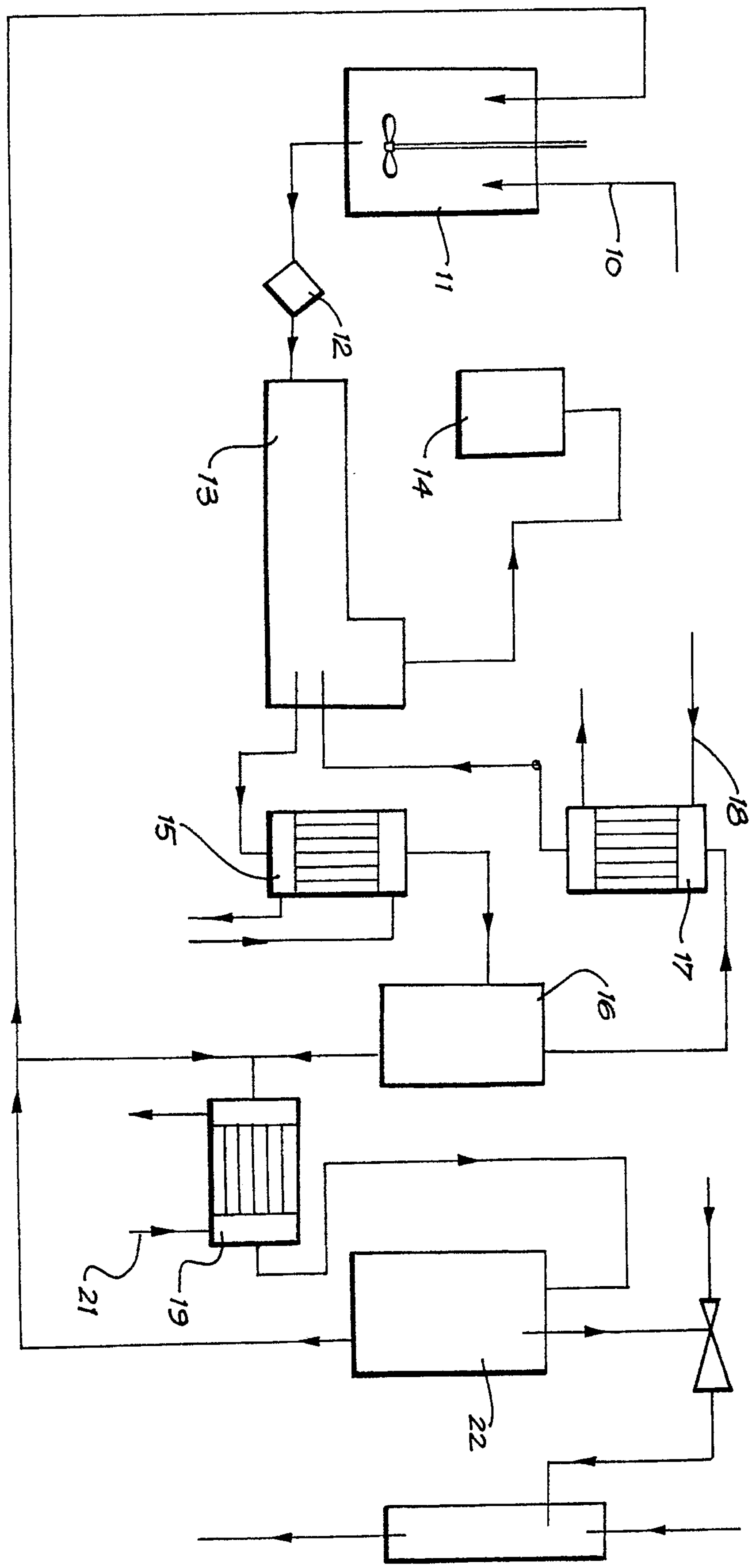
10
11
12
13
14
15
16
17
18
19
21
22